Europäisches Patentamt

European Patent Office

Office européen des brevets

(11) Publication number: **0 217 379 B1**

## EUROPEAN PATENT SPECIFICATION

(45) Date of publication of patent specification: **17.07.91**

(51) Int. Cl.⁵: **A61K 37/54**, //C12N9/64, C12N15/00

(21) Application number: **86113507.7**

(22) Date of filing: **01.10.86**

(54) **Thrombolytic composition and a process for production thereof.**

(30) Priority: **02.10.85 JP 219606/85**

(43) Date of publication of application:
**08.04.87 Bulletin 87/15**

(45) Publication of the grant of the patent:
**17.07.91 Bulletin 91/29**

(84) Designated Contracting States:
**AT BE CH DE FR GB IT LI LU NL SE**

(56) References cited:
**EP-A- 0 218 112**
**EP-A- 0 228 862**
**GB-A- 985 498**

(73) Proprietor: **MOCHIDA PHARMACEUTICAL CO., LTD.**
**7, Yotsuya 1-chome**
**Shinjuku-ku Tokyo 160(JP)**

(72) Inventor: **Ichimura, Michio**
**5-43, Takasu**
**Fujieda-shi Shizuoka-ken(JP)**
Inventor: **Ohta, Hidemi**
**5-2-16, Ohsu**
**Fujieda-shi Shizuoka-ken(JP)**
Inventor: **Sakai, Kiyoshi**
**1-10-15, Takasu**
**Fujieda-shi Shizuoka-ken(JP)**
Inventor: **Kunihiro, Yasuyuki**
**3-10-2, Takaoka**
**Fujieda-shi Shizuoka-ken(JP)**
Inventor: **Nakashiro, Takehisa**
**4-7-21, Takaoka**
**Fujieda-shi Shizuoka-ken(JP)**

(74) Representative: **Hoffmann, Klaus, Dr. rer. nat. et al**
**Hoffmann . Eitle & Partner Patentanwälte**
**Postfach 81 04 20 Arabellastrasse 4**
**W-8000 München 81(DE)**

**Description**

The present invention relates to a thrombolytic composition and a process for the production thereof.

Thrombolytic compositions have been hitherto developed from urokinase isolated and purified from urine or cultured mammalian kidney cells. Also, streptokinase extracted from β-hemolytic streptococcus has been provided for practical use. However, urokinase has a poor affinity to thrombus so that a large dose of urokinase is required to achieve the desired therapeutic effects. Particularly with systemic administration, bleeding has been feared due to destruction of coagulation factors by large quantities of plasmin induced in the blood. Tissue plasminogen activator (hereafter referred to as t-PA) found in human or other animal tissue or tissue culture medium derived from human or other animal tissues or tumor cells has a higher affinity to thrombus and a higher thrombolytic activity as compared to urokinase. Therefore, t-PA has been expected to give desired therapeutic effects by administration in a lesser dose and as a new thrombolytic agent. In addition, it has recently been attempted to produce t-PA by genetic engineering techniques.

The present inventors have purified t-PA and investigated a process for producing a thrombolytic composition comprising t-PA as an effective component. However, it has been clarified that the solubility of t-PA decreases as the degree of purification of t-PA becomes higher, and such is a serious obstacle to medical preparations.

From GB-A-985 498 a plasmin preparation is known comprising plasmin and an amino acid as a stabiliser and solubiliser thereof.

The present invention is directed to a thrombolytic composition which includes arginine or an acid addition salt thereof to increase the solubility of t-PA, and a process for the production thereof.

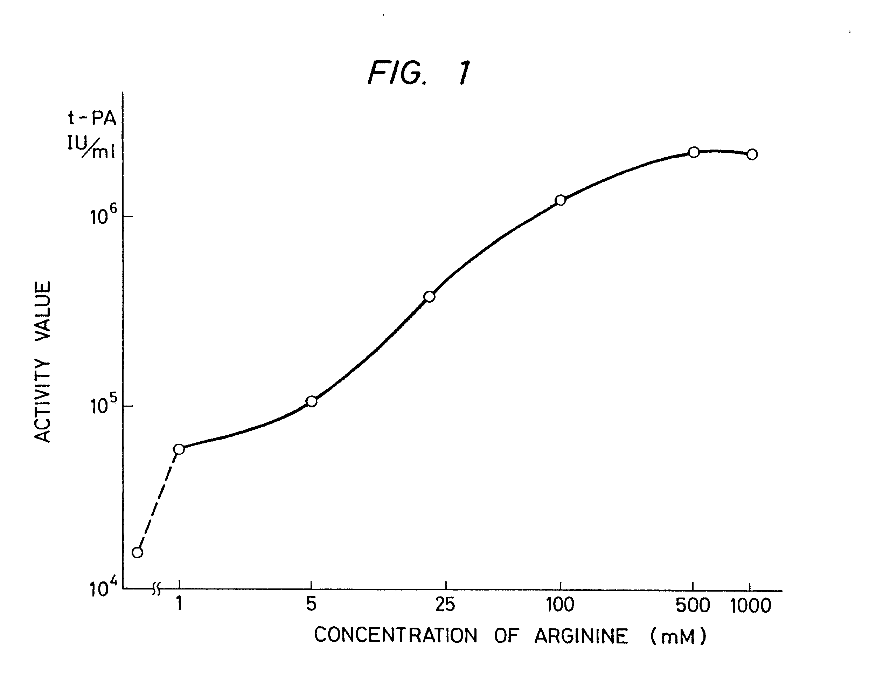

Figure 1 is a graph showing the relationship between the solubility of t-PA and concentration of arginine.

The present inventors have found that the solubility of t-PA is markedly increased by the use of a solvent system containing arginine or an acid addition salt thereof.

Arginine used in the present invention can be any of D-, L- and racemic-form and may further be an acid addition salt thereof, e.g. the hydrochloride (hereafter the term arginine includes all of the above, unless otherwise indicated). The amount of arginine necessary for enhancing the solubility of t-PA is 1 mM to 500 mM, preferably 5 mM to 200 mM. Even when the amount exceeds 500 mM, the system is effective but an increase in the solubility of t-PA corresponding to the increase in the amount of arginine is not expected. Therefore, an amount of 500 mM or less is most practical. Further, it is more preferred to use neutral salts, especially sodium chloride in a concentration of 0.02 M to 2.0 M, preferably 0.1 to 1.0 M, in combination with arginine. It is preferred that the pH of a t-PA solution containing arginine or arginine and neutral salts such as sodium chloride, be maintained in a range of 2 to 12, more preferably 6 to 11, by adding buffer solution of, e.g. sodium phosphate.

The thrombolytic composition of the present invention comprises at least arginine and t-PA as the effective components. Other components, e.g. excipients, stabilizers, conventionally used in medical preparations, for example albumin, mannitol, gelatin and sodium chloride, may be optionally incorporated.

The thrombolytic composition of the present invention can be prepared by dissolving purified t-PA in a solvent containing arginine and then packing the sterilized solution e.g. in an ampule, a vial. If necessary, freeze-drying can be performed. Instead of dissolving t-PA in a solvent containing arginine, t-PA and a determined amount of arginine may be weighed and dissolved in a suitable solvent or packed in a suitable container, if necessary, together with other components, to prepare the thrombolytic composition. The solvents used in the present invention include distilled water for injection, saline, 0.01 M to 0.1 M phosphate buffer, and other pharmaceutically acceptable solvents. As the medical preparation, an injection is generally advantageous, but as long as the thrombolytic activity of t-PA can be maintained, any medical preparation can be used. The dose of the thrombolytic composition according to the present invention is generally 20,000 to 50,000 IU/kg for an adult, but can be appropriately varied depending upon conditions.

As stated above, by using the solvent containing arginine, it is possible to prepare a t-PA solution of high concentration. Consequently, it is possible to pack large quantities of t-PA in a container of a small volume, e.g. a vial or an ampule, and thus a therapeutically effective thrombolytic composition can be produced. For example, as apparent from Table 1 described below, the solubility limit of t-PA in saline is around 15,000 IU/ml, so that it is difficult even to produce preparations of 100,000 IU/vial. However, when the solvent is saline containing arginine hydrochloride, it is easy to produce preparations of around 2,000,000-5,000,000 IU/vial. Accordingly, it is extremely useful in medical preparation of t-PA to incorporate arginine in the t-PA solution.

The present invention will be described in more detail with reference to experiments and examples. The following experiments and examples use only t-PA purified from culture medium of human melanoma cells

2

and Chinese hamster ovary (hereafter referred to as CHO) cells to which t-PA gene was transferred by a genetic engineering technique, but t-PA may be any of t-PA derived from human or other animal tissue, t-PA purified from a cell culture derived from human or other animal tissue, t-PA obtained by genetic engineering techniques other than the aforesaid techniques, such as t-PA obtained for example from microorganisms such as t-PA transferred eucaryocyte, Escherichia coli, Bacillus subtilis and yeast, to which the t-PA gene is transferred.

The measurement of the activity of t-PA was performed by the fibrin plate method using 95% clottable fibrinogen (plasminogen content, ca. 50 casein unit/g clottable protein), using t-PA (approved by WHO) as a standard.

## Preparation 1 of t-PA

60 ℓ of a crude t-PA culture medium produced from a human melanoma cell culture in accordance with the method of D. Collen et al. (The Journal of Biological Chemistry, 256(13), 7035-7041, 1981) were purified with reference to the method of D.C. Rijken et al. (Thromb Haemosta, 48(3), 294-296, 1982). Namely, after purification by Zn-chelate chromatography (13.5 x 17.5 cm), the culture medium was further applied to Concanavalin A-Sepharose chromatography (5 x 30 cm). t-PA was eluted with a 2.0 M potassium thiocyanate solution containing 0.4 M $\alpha$-D-methylmanoside from the Concanavalin A-Sepharose column. After concentrating the eluted t-PA with polyethylene glycol, gel filtration was performed through a column (7.5 x 90 cm) packed with Sephacryl® S 200 (made by Pharmacia) equilibrated with a 0.01 M phosphate buffer (pH 7.5) containing 0.25 M arginine hydrochloride to give purified t-PA. 18,000,000 IU of purified t-PA showing a specific activity of $2.5 \times 10^5$ IU/mg was obtained by the said purification steps. The thus obtained t-PA was dialyzed to distilled water at 4°C overnight and then freeze-dried to provide the following experiments and examples.

## Preparation 2 of t-PA

A t-PA gene was prepared according to the method of D. Pennica et al. (Nature, 301(20), 214-221, 1983). The aforesaid t-PA gene was transferred to CHO cells and then the CHO cells were cultured according to the method of R.J. Kaufman and P.A. Sharp (Journal of Molecular Biology, 159, 601-621, 1982). Thereafter, 40 l of crude t-PA culture medium were obtained and said medium was purified in a manner similar to Preparation 1 to give 12,000,000 IU of purified t-PA showing a specific activity of $1.7 \times 10^5$ IU/mg.

## Experiment 1

The influence of the arginine concentration on the solubility of t-PA was examined. Five mg each of the purified t-PA obtained in Preparation 1 were weighed and dissolved in 0.5 ml of a solution of arginine hydrochloride having a concentration as shown in Table 1. The activity of t-PA in the solution was measured to examine the solubility of t-PA. In case the t-PA was not completely dissolved but precipitates were formed, the activity in the supernatant was measured. The results are shown in Table 1 and Figure 1.

EP 0 217 379 B1

Table 1

| Solvent System | Titer of Solution (supernatant) | Dissolved State |
|---|---|---|
| Saline | 16,100 IU/ml | white turbid |
| Saline containing: | | |
| 1 mM L-arginine hydrochloride | 57,000 IU/ml | white turbid |
| 5 mM L-arginine hydrochloride | 104,000 IU/ml | white turbid |
| 25 mM L-arginine hydrochloride | 372,000 IU/ml | white turbid |
| 100 mM L-arginine hydrochloride | 1,220,000 IU/ml | slightly white turbid |
| 500 mM L-arginine hydrochloride | 2,270,000 IU/ml | clear |
| 1,000 mM L-arginine hydrochloride | 2,200,000 IU/ml | clear |

Experiment 2

The influence of arginine on the solubility of t-PA was examined. One mg each of the t-PA purified in Preparations 1 and 2 was dissolved in 0.5 ml of a solution containing 25 mM of various forms of arginine and the activity of t-PA in the solutions was measured in a manner similar to Experiment 1. The results are shown in Table 2.

4

Table 2

| Solvent System | t-PA in Preparation 1 Titer of Solution (supernatant) | Dissolved State | t-PA in Preparation 2 Titer of Solution (supernatant) | Dissolved State |
|---|---|---|---|---|
| Distilled water containing: | | | | |
| 25 mM L-arginine | 306,000 IU/ml | almost clear | 246,000 IU/ml | almost clear |
| 25 mM L-arginine hydrochloride | 351,000 IU/ml | almost clear | 254,000 IU/ml | almost clear |
| 25 mM D-arginine hydrochloride | 298,000 IU/ml | almost clear | 225,000 IU/ml | almost clear |
| 25 mM DL-arginine hydrochloride | 314,000 IU/ml | almost clear | 241,000 IU/ml | almost clear |
| 25 mM D-arginine | 245,000 IU/ml | almost clear | 218,000 IU/ml | almost clear |

Experiment 3

The solubility of t-PA in a solvent system containing L-arginine hydrochloride and sodium chloride was examined. In 0.5 ml of a solution containing L-arginine hydrochloride or combined L-arginine hydrochloride and sodium chloride, the pH of which had been adjusted, 1 mg or 5 mg of purified t-PA was dissolved. The activity of t-PA in the solutions was measured to examine the solubility of t-PA. The results are shown in Tables 3 and 4.

Table 3

| Solvent System | Titer of Solution (supernatant) | Dissolved State |
|---|---|---|
| Distilled water containing 100 mM L-arginine hydrochloride, pH 7.0 | 1,220,100 IU/ml | slightly white turbid |
| 0.01 M phosphate buffer containing 100 mM L-arginine hydrochloride and 0.14 M NaCl, pH 7.0 | 1,640,000 IU/ml | clear |
| 0.01 M phosphate buffer containing 100 mM L-arginine hydrochloride and 0.30 M NaCl, pH 7.0 | 2,090,000 IU/ml | clear |

Purified t-PA weighed: 5 mg

Amount of solvent: 0.5 ml

Table 4

| Solvent System | Titer of Solution (supernatant) | Dissolved State |
|---|---|---|
| Distilled water containing 25 mM L-arginine hydrochloride and 0.14 M NaCl, pH 2.0 (adjusted with HCl) | 187,000 IU/ml | slightly white turbid |
| Distilled water containing 25 mM L-arginine hydrochloride and 0.14 M NaCl, pH 4.0 (adjusted with citric acid) | 291,000 IU/ml | white turbid |
| 0.05 M phosphate buffer containing 25 mM L-arginine hydrochloride and 0.14 M NaCl, pH 7.0 | 414,000 IU/ml | clear |
| Distilled water containing 25 mM L-arginine hydrochloride and 0.14 M NaCl, pH 9.0 (adjusted with NaOH) | 396,000 IU/ml | clear |
| Distilled water containing 25 mM L-arginine hydrochloride and 0.14 M NaCl, pH 11.0 (adjusted with NaOH) | 407,000 IU/ml | clear |

Purified t-PA weighed:   1 mg

Amount of solvent:   0.5 ml


Experiment 4 General safety and pyrogen test

The medical preparations prepared in Example 1 and Example 2 below were dissolved in saline or distilled water for injection and each solution was given to mice and guinea pigs at a dose of 500,000 IU/kg. No abnormality was observed in any of the mice or guinea pigs. 200,000 IU/kg of the same preparations were administered to rabbits to perform a pyrogen test. All of the preparations were negative.

As is evident from the foregoing experiments, arginine is useful for enhancing the solubility of t-PA.

7

Example 1

| | |
|---|---|
| t-PA | 5,000,000 IU |
| L-arginine hydrochloride | 21 mg |
| Sodium phosphate | 173.9 mg |
| Purified gelatin | 100 mg |

Each component described above was dissolved in 10 ml of distilled water for injection. After aseptic filtration, 0.1 ml of each was packed in a vial and freeze-dried to prepare a thrombolytic composition.

Example 2

| | |
|---|---|
| t-PA | 5,000,000 IU |
| L-arginine hydrochloride | 52.5 mg |
| Sodium phosphate | 173.9 mg |
| Sodium chloride | 64.3 mg |
| Human serum albumin | 20 mg |

Each component described above was weighed and a thrombolytic composition was prepared in a manner similar to Example 1.

**Claims**

1. A pharmaceutical composition comprising tissue plasminogen activator and at least one member selected from arginine and an acid addition salt of arginine for use as a thrombolytic agent.

2. A pharmaceutical composition as claimed in claim 1, wherein said composition further comprises one or more member selected from sodium chloride, sodium phosphate, gelatin, mannitol and human serum albumin.

3. A pharmaceutical composition as claimed in claim 1 or 2, wherein said arginine is selected from D-arginine, L-arginine, and racemic-form arginine.

4. A pharmaceutical composition as claimed in claim 1 or 2, wherein said acid addition salt of arginine is arginine hydrochloride.

5. A pharmaceutical composition as claimed in claim 1 or 2, wherein said member is contained in an amount of 1 mM to 500 mM.

6. A pharmaceutical composition as claimed in claim 1 or 2, wherein said tissue plasminogen activator is one produced by extraction and purification from human or other animal tissue or from a cell culture medium derived from human or other animal tissue.

7. A pharmaceutical composition as claimed in claim 1 or 2, wherein tissue plasminogen activator is one produced by extraction and purification from a culture medium of human melanoma cells.

8. A pharmaceutical composition as claimed in claim 1 or 2, wherein said tissue plasminogen activator is

8

EP 0 217 379 B1

one produced by culturing Chinese hamster ovary cells to which a tissue plasminogen activator gene has been transferred.

9. A pharmaceutical composition as claimed in claim 1 or 2, wherein said tissue plasminogen activator is one produced by culturing a microorganism selected from eucaryocyte, Escherichia coli, Bacillus subtilis and yeast, to which tissue plasminogen activator gene has been transferred.

10. A pharmaceutical composition as claimed in claim 1 or 2, said composition being a unit dosage form of a freeze-dried preparation.

11. A process for producing a pharmaceutical composition, comprising packing a tissue plasminogen activator in a container for medical preparation in the presence of arginine or an acid addition salt of arginine.

12. The use of tissue plasminogen activator and at least one member selected from arginine and an acid addition salt of arginine to prepare a pharmaceutical for the treatment of thrombosis.

**Claims for the following Contracting State: AT**

1. A process for preparing a pharmaceutical composition for use as thrombolytic agent, characterized by mixing a tissue plasminogen activator and at least one member selected from arginine and an acid addition salt of arginine.

2. The process according to claim 1, characterized by further mixing one or more member selected from sodium chloride, sodium phosphate, gelatine, mannitol and human serum albumin.

3. The process according to claim 1 or 2, wherein said arginine is selected from D-arginine, L-arginine, and racemic-form argenine.

4. The process according to claim 1 or 2, wherein said acid addition salt of arginine is arginine hydrochloride.

5. The process according to claim 1 or 2, wherein said member is contained in an amount of 1 mM to 500 mM.

6. The process according to claim 1 or 2, wherein said tissue plasminogen activator is one produced by extraction and purification from human or other animal tissue or from a cell culture medium derived from human or other animal tissue.

7. The process according to claim 1 or 2, wherein said tissue plasmimogen activator is one produced by extraction and purification from a culture medium of human melanoma cells.

8. The process according to claim 1 or 2, wherein said tissue plasminogen activator is one produced by culturing Chinese hamster ovary cells to which a tissue plasminogen activator gene has been transferred.

9. The process according to claim 1 or 2, wherein said tissue plasminogen activator is one produced by culturing a microorganism selected from eucaryocyte, Escherichia coli, Bacillus subtilis and yeast, to which tissue plasminogen activator gene has been transferred.

10. The use of tissue plasminogen activator and at least one member selected from arginine and an acid addition salt of arginine to prepare a pharmaceutical for the treatment of thrombosis.

**Revendications**

1. Composition pharmaceutique comprenant de l'activateur du plasminogène tissulaire et au moins un élément choisi parmi l'arginine et un sel d'addition d'acide de l'arginine utilisable comme agent thrombolytique.

9

2. Composition pharmaceutique selon la revendication 1, dans laquelle ladite composition comprend en outre un ou plusieurs éléments choisis parmi le chlorure de sodium, le phosphate de sodium, la gélatine, le mannitol et la sérum albumine humaine.

3. Composition pharmaceutique selon la revendication 1 ou 2, dans laquelle ladite arginine est choisie parmi la D-arginine, la L-arginine et l'arginine sous forme racémique.

4. Composition pharmaceutique selon la revendication 1 ou 2, dans laquelle ledit sel d'addition d'acide de l'arginine est le chlorhydrate d'arginine.

5. Composition pharmaceutique selon la revendication 1 ou 2, dans laquelle ledit élément est contenu en une quantité de 1 mM à 500 mM.

6. Composition pharmaceutique selon la revendication 1 ou 2, dans laquelle ledit activateur du plasminogène tissulaire est un activateur produit par extraction et purification à partir de tissu humain ou d'un autre animal ou à partir d'un milieu de culture de cellules dérivé de tissu humain ou d'un autre animal.

7. Composition pharmaceutique selon la revendication 1 ou 2, dans laquelle l'activateur du plasminogène tissulaire est un activateur produit par extraction et purification à partir d'un milieu de culture de cellules de mélanome humain.

8. Composition pharmaceutique selon la revendication 1 ou 2, dans laquelle ledit activateur du plasminogène tissulaire est un activateur produit par culture de cellules ovariennes de hamster chinois auxquelles un gène d'activateur du plasminogène tissulaire a été transféré.

9. Composition pharmaceutique selon la revendication 1 ou 2, dans laquelle ledit activateur du plasminogène tissulaire est un activateur produit par culture d'un micro-organisme choisi parmi un eucaryocyte, Escherichia coli, Bacillus subtilis et une levure, auquel un gène d'activateur du plasminogène tissulaire a été transféré.

10. Composition pharmaceutique selon la revendication 1 ou 2, ladite composition étant sous forme de dose unitaire d'une préparation lyophilisée.

11. Procédé de préparation d'une composition pharmaceutique, comprenant le conditionnement d'un activateur du plasminogène tissulaire dans un récipient pour préparation médicale en présence d'arginine ou d'un sel d'addition d'acide de l'arginine.

12. Utilisation d'un activateur du plasminogène tissulaire et d'au moins un élément choisi parmi l'arginine et un sel d'addition d'acide de l'arginine pour préparer une composition pharmaceutique pour le traitement de la thrombose.

**Revendications pour l'Etat contractant suivant: AT**

1. Procédé de préparation d'une composition pharmaceutique utilisable comme agent thrombolytique, caractérisé en ce que l'on mélange un activateur du plasminogène tissulaire et au moins un élément choisi parmi l'arginine et un sel d'addition d'acide de l'arginine.

2. Procédé selon la revendication 1, caractérisé en ce que l'on mélange en outre un ou plusieurs éléments choisis parmi le chlorure de sodium, le phosphate de sodium, la gélatine, le mannitol et la sérum albumine humaine.

3. Procédé selon la revendication 1 ou 2, dans lequel ladite arginine est choisie parmi la D-arginine, la L-arginine et l'arginine sous forme racémique.

4. Procédé selon la revendication 1 ou 2, dans lequel ledit sel d'addition d'acide de l'arginine est le chlorhydrate d'arginine.

5. Procédé selon la revendication 1 ou 2, dans lequel ledit élément est contenu en une quantité de 1 mM à 500 mM.

6. Procédé selon la revendication 1 ou 2, dans lequel ledit activateur du plasminogène tissulaire est un activateur produit par extraction et purification à partir de tissu humain ou d'un autre animal ou à partir d'un milieu de culture de cellules dérivé de tissu humain ou d'un autre animal.

7. Procédé selon la revendication 1 ou 2, dans lequel ledit activateur du plasminogène tissulaire est un activateur produit par extraction et purification à partir d'un milieu de culture de cellules de mélanome humain.

8. Procédé selon la revendication 1 ou 2, dans lequel ledit activateur du plasminogène tissulaire est un activateur produit par culture de cellules ovariennes de hamster chinois auxquelles un gène d'activateur du plasminogène tissulaire a été transféré.

9. Procédé selon la revendication 1 ou 2, dans lequel ledit activateur du plasminogène tissulaire est un activateur produit par culture d'un micro-organisme choisi parmi un eucaryocyte, Escherichia coli, Bacillus subtilis et une levure, auquel un gène d'activateur du plasminogène tissulaire a été transféré.

10. Utilisation d'un activateur du plasminogène tissulaire et d'au moins un élément choisi parmi l'arginine et un sel d'addition d'acide de l'arginine pour préparer une composition pharmaceutique pour le traitement de la thrombose.

**Patentansprüche**

1. Pharmazeutische Zusammensetzung, dadurch gekennzeichnet, daß sie Gewebe-Plasminogenaktivator und zumindest eine Komponente, ausgewählt aus Arginin und einem Säureadditionssalz von Arginin enthält, zur Verwendung als thrombolytisches Mittel.

2. Pharmazeutische Zusammensetzung nach Anspruch 1, dadurch gekennzeichnet, daß die Zusammensetzung weiterhin eine oder mehrere Komponenten enthält, die aus Natriumchlorid, Natriumphosphat, Gelatine, Mannit und Menschenserumalbumin ausgewählt ist.

3. Pharmazeutische Zusammensetzung nach Anspruch 1 oder 2, dadurch gekennzeichnet, daß das Arginin ausgewählt ist aus D-Arginin, L-Arginin und der razemischen Form von Arginin.

4. Pharmazeutische Zusammensetzung nach Anspruch 1 oder 2, dadurch gekennzeichnet, daß das säureadditionssalz von Arginin Argininhydrochlorid ist.

5. Pharmazeutische Zusammensetzung nach Anspruch 1 oder 2, dadurch gekennzeichnet, daß die Komponente in einer Menge von 1 mM bis 500 mM enthalten ist.

6. Pharmazeutische Zusammensetzung nach Anspruch 1 oder 2, dadurch gekennzeichnet, daß der Gewebe-Plasminogenaktivator durch Extraktion und Reinigung aus menschlichem oder anderem tierischem Gewebe oder aus einem Zellkulturmedium gewonnen ist, welches sich von menschlichem oder anderem tierischem Gewebe ableitet.

7. Pharmazeutische Zusammensetzung nach Anspruch 1 oder 2, dadurch gekennzeichnet, daß der Gewebe-Plasminogenaktivator durch Extraktion und Reinigung aus einem Kulturmedium von menschlichen Melanomzellen hergestellt ist.

8. Pharmazeutische Zusammensetzung nach Anspruch 1 oder 2, dadurch gekennzeichnet, daß der Gewebe-plasminogenaktivator durch Züchten von Ovarialzellen von chinesischen Hamstern hergestellt ist, in welche ein Gen des Gewebe-Plasminogenaktivators übertragen worden ist.

9. Pharmazeutische Zusammensetzung nach Anspruch 1 oder 2, dadurch gekennzeichnet, daß der Gewebe-Plasminogenaktivator durch Züchten eines Mikroorganismus hergestellt ist, der ausgewählt ist aus Eucaryot, Escherichia coli, Bacillus subtilus und Hefe, in welche ein Gewebe-Plasminogenaktivatorgen übertragen worden war.

EP 0 217 379 B1

10. Pharmazeutische Zusammensetzung nach Anspruch 1 oder 2, dadurch gekennzeichnet, daß die Zusammensetzung eine Einheitsdosisform einer gefriergetrockneten Präparation ist.

11. Verfahren zur Herstellung einer pharmazeutischen Zusammensetzung, gekennzeichnet durch Abpacken eines Gewebe-plasminogenaktivators in einen Behälter für ein medizinisches Präparat in der Gegenwart von Arginin oder einem Säureadditionssalz von Arginin.

12. Verwendung von Gewebe-Plasminogenaktivator und zumindest einer Komponente, ausgewählt aus Arginin und einem Säureadditionssalz von Arginin, zur Herstellung eines pharmazeutischen Mittels zur Behandlung von Thrombose.

**Patentansprüche für folgenden Vertragsstaat: AT**

1. Verfahren zur Herstellung einer pharmazeutischen Zusammensetzung zur Verwendung als thrombolytisches Mittel, gekennzeichnet durch Mischen eines Gewebe-Plasminogenaktivators und zumindest einer Komponente, ausgewählt aus Arginin und einem Säureadditionssalz von Arginin.

2. Verfahren nach Anspruch 1, dadurch gekennzeichnet, daß weiterhin eine oder mehrere Komponenten, ausgewählt aus Natriumchlorid, Natriumphosphat, Gelatine, Mannit und Menschenserumalbumin gemischt werden.

3. Verfahren nach Anspruch 1 oder 2, dadurch gekennzeichnet, daß Arginin ausgewählt ist aus D-Arginin, L-Arginin und der razemischen Form von Arginin.

4. Verfahren nach Anspruch 1 oder 2, dadurch gekennzeichnet, daß das säureadditionssalz von Arginin Argininhydrochlorid ist.

5. Verfahren nach Anspruch 1 oder 2, dadurch gekennzeichnet, daß die Komponente in einer Menge von 1 mM bis 500 mM enthalten ist.

6. Verfahren nach Anspruch 1 oder 2, dadurch gekennzeichnet, daß der Gewebe-Plasminogenaktivator durch Extraktion und Reinigung von menschlichem oder anderem tierischem Gewebe oder von einem Zellkulturmedium hergestellt ist, welches sich von menschlichem oder anderem tierischem Gewebe ableitet.

7. Verfahren nach Anspruch 1 oder 2, dadurch gekennzeichnet, daß der Gewebe-Plasminogenaktivator durch Extraktion und Reinigung von einem Kulturmedium von menschlichen Melanomzellen hergestellt wird.

8. Verfahren nach Anspruch 1 oder 2, dadurch gekennzeichnet, daß der Gewebe-Plasminogenaktivator durch Züchten von Ovarialzellen von chinesischen Hamstern hergestellt wird, welchen ein Gewebe-Plasminogenaktivatorgen übertragen worden ist.

9. Verfahren nach Anspruch 1 oder 2, dadurch gekennzeichnet, daß der Gewebe-Plasminogenaktivator durch Züchten eines Mikroorganismus hergestellt ist, der ausgewählt ist aus Eucaryot, Escherichia Coli, Bacillus subtilus und Hefe, denen Gewebe-Plasminogenaktivator-Gen übertragen worden ist.

10. Verwendung des Gewebe-Plasminogenaktivators und zumindest einer Komponente ausgewählt aus Arginin und einem Säureadditionssalz von Arginin zur Herstellung einer pharmazeutischen Zusammensetzung zur Behandlung von Thrombose.

FIG. 1